(19) Europäisches Patentamt / European Patent Office / Office européen des brevets

(11) **EP 4 385 406 A2**

(12) **EUROPEAN PATENT APPLICATION**

(43) Date of publication:
**19.06.2024   Bulletin 2024/25**

(21) Application number: **24173748.5**

(22) Date of filing: **15.10.2020**

(51) International Patent Classification (IPC):
**A61B 5/107** (2006.01)

(52) Cooperative Patent Classification (CPC):
**A61B 5/6824; A61B 5/1071; A61B 5/6828;
A61B 5/6829;** A61B 5/6832; A61B 5/7246;
A61B 2505/09

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.10.2019   GB 201915139**

(62) Document number(s) of the earlier application(s) in
accordance with Art. 76 EPC:
**20797863.6 / 4 044 904**

(71) Applicant: **McLaren Applied Limited
Woking
Surrey GU21 6JD (GB)**

(72) Inventors:
• **Gaskell, Matthew Alastair
London, N19 4NN (GB)**
• **Reddall, Nicholas Henry
London, SW18 5UF (GB)**

(74) Representative: **Schott, Jakob Valentin
Wuesthoff & Wuesthoff
Patentanwälte und Rechtsanwalt PartG mbB
Schweigerstraße 2
81541 München (DE)**

Remarks:
This application was filed on 02.05.2024 as a
divisional application to the application mentioned
under INID code 62.

(54) **SENSOR DETERMINATION**

(57)   Disclosed is a method for determining an unworn state of a sensor system comprising a pair of sensors configured for mounting on first and second body parts either side of a joint. The method comprises: obtaining one or more measurements from each sensor; calculating a relative angle between the two sensors using one or more of the obtained one or more measurements; calculating, using one or more of the obtained one or more measurements, a joint angle between the first and second body parts, where the joint angle is defined in a plane of normal bending of the joint; and determining, based on the calculated angles, whether either of the sensors is not mounted and if so, determining that the system is in an unworn state. Also disclosed is a system for providing information about a joint.

**FIG. 3**

EP 4 385 406 A2

**Description**

CROSS-REFERENCE TO RELATED APPLICATIONS

This application claims the benefit of priority from United Kingdom patent application no. 1915139.8, filed October 18, 2019, which is incorporated by reference herein in its entirety.

FIELD

This invention relates to a method for determining an unworn state of a system for providing information about a joint.

BACKGROUND

There is a growing popularity for devices that measure movement. These sensing devices can be in the form of wearable devices that measure movement of a user, a smartphone that is carried by the user to measure movement of the user or moveable devices that can generally sense movement, for instance video game controllers or sensors attached to industrial equipment. In particular, wearable devices can be utilised to track motion of a human or other animal and, in particular can be used to monitor the motion of a specific joint.

These sensing devices may include a satellite positioning sensor which can sense the location of the device, and one or more motion sensors which sense motion and/or orientation of the device. These motion sensors may include one or more of an accelerometer, a gyroscope, a magnetometer, a compass and a barometer. Measurements taken by the sensors can be used to provide information about the joint.

When using a wearable device, it may be necessary for the device to be used for extended periods of time such as a month or more so as to build up data which changes only slowly over time. This means that any sensing device which is used will likely need to be removed for any number of reasons including, but not limited to, the need to recharge a power supply on the device, the desire to clean the sensor so as to remove a build-up of dirt and grime or spillages thereon, or to wash the part of the person or animal on which the sensor is mounted. Such a sensing device could also fall off. If measurements taken by the device when it has been removed or otherwise dislodged from its intended location are included when providing the information, this could be misleading as regards monitoring the joint in question.

Thus, it would be desirable for there to be improvements in how wearable sensors are operated.

SUMMARY

According to a first aspect of the present disclosure, there is provided a method for determining an unworn state of a sensor system comprising a pair of sensors configured for mounting on first and second body parts either side of a joint, the method comprising: obtaining one or more measurements from each sensor; calculating a relative angle between the two sensors using one or more of the obtained one or more measurements; calculating, using one or more of the obtained one or more measurements, a joint angle between the first and second body parts, where the joint angle is defined in a plane of normal bending of the joint; determining, based on the calculated angles, whether either or both of the sensors is not mounted and if so, determining that the system is in an unworn state.

In some variations, obtaining one or more measurements from each sensor may be performed multiple times in a time period and the calculating a relative angle and the calculating a joint angle may be performed using measurements obtained at at least some of the multiple times. The method may include the further step of, following a determination that the sensor system is in an unworn state, adopting a different procedure with regard to calculating the joint angle at subsequent times. The different procedure can comprise any one or more of: not calculating the joint angle at subsequent times; differently processing the calculated joint angles at subsequent times from those calculated at times prior to the determination; not storing the joint angles calculated at subsequent times.

The method may further comprise correlating the joint angles and relative angles calculated at multiple times to provide information about the joint over the time period, and adopting a different procedure may comprise omitting any data calculated using measurements made at subsequent times from the information. The information may comprise any one or more of: joint angle variation during the time period; duration of time in which the joint is active; duration of time in which the joint is bearing a load; length of the time period; and if the joint is a knee, step count over the time period.

Adopting a different procedure can comprise performing calculations to provide different information from the information about the joint, wherein the different information includes any one or more of: unworn time; and if the first sensor is determined to be charging, charging time.

An unworn state may include any one or more of: the first sensor having been at least partially removed from its mounted position; the first sensor falling off its mounted position at least to some extent; the first sensor being switched on but not mounted on the first body part; the second sensor being switched on but not mounted on the second body part.

The determining if the first sensor is mounted on the first body part may comprise calculating a value of a function of the calculated angles. The method may further comprise processing multiple calculated values of the function over a time period to determine a moving average value for the function and wherein the determining may further comprise comparing the determined moving average value to a threshold value. The function can comprise a function of a joint angle penalty and a relative angle penalty. In some implementations, if the joint angle is within a feasible range for the joint, the joint angle penalty may be zero and if the joint angle is not within a feasible range for the joint, the joint angle penalty may have a value which depends on how far outside the feasible range the joint angle is. In some implementations, if the relative angle is less than or equal to a threshold relative value, the relative angle penalty may be zero and if the relative angle penalty is greater than the threshold relative value, the relative angle penalty may depend on how much greater the relative angle is than the threshold relative value.

The relative angle may be a difference in tilt angle of the two body parts, where tilting occurs about an axis perpendicular or substantially perpendicular to the plane in which the joint angle is defined.

The joint angle can be defined in an x-z plane and the tilt angle can be defined in the x-y plane and may be non-zero if the first and second body parts undergo a relative roll about the x-axis.

The method may further comprise, prior to the obtaining, determining whether the first and second sensors are mounted on correct respective first and second body parts and/or in substantially a predetermined orientation relative to the correct respective first and second body parts, and if one or both sensors is determined to be mounted on the wrong body part or in a different orientation from the predetermined orientation, adjusting the calculating steps to take account of an actual determined mounting location and/or orientation.

The method may further comprise, prior to the obtaining, calibrating the first and second sensors with respect to a predetermined orientation relative to the respective first and second body parts.

The joint may be a knee or an elbow. The joint could be other joints such as a shoulder or ankle joint.

Any of the above methods and features can be used in any feasible combination.

According to a second aspect of the invention, there is provided a system for providing information about a joint, comprising: a first sensor unit configured to be mounted on a first body part on a first side of a joint, the first sensor unit comprising: one or more first sensors arranged to take one or more measurements relating to the first body part and one or more measurements relating to the first sensor; and a transmitter arranged to transmit taken measurements; and a second, master sensor unit configured to be mounted on a second body part on a second side of a joint, the second master sensor unit comprising: one or more second sensors arranged to take one or more measurements relating to the second body part and one or more measurements relating to the second sensor; a receiver arranged to receive measurements transmitted from the first sensor unit transmitter; and a calculation unit configured to calculate, using one or more of the measurements, a relative angle between the two sensors and a joint angle between the first and second body parts, where the joint angle is defined in a plane of normal bending of the joint, the calculation unit being further configured to determine, based on the calculated angles, whether either or both of the sensor units is not mounted and if so, determine that the system is in an unworn state.

The first and second sensor units may be configured to take measurements multiple times in a time period and the calculation unit may be configured to calculate a relative angle and a joint angle using measurements obtained at each of at least some of the multiple times. The calculation unit may be further configured to, following a determination that the system is in an unworn state, adopt a different procedure with regard to calculating the joint angle at subsequent times. The calculation unit may be further configured to correlate the joint angles and relative angles calculated at multiple times to provide information about the joint over the time period, and in some implementations, adopting a different procedure may comprise omitting any data relating to measurements made at subsequent times from the information.

The second, master sensor unit may be configured to provide the information to a mobile device for viewing by a user. The information may be for use in assessing health and/or rehabilitation of the joint.

In some examples, the first and second sensor units may be generally planar having front and back faces and may be configured for their back faces to be mounted on the first and second body parts at a side of the joint substantially parallel to the plane of normal bending of the joint.

Any of the above features may be used in any feasible combination.

DRAWINGS

The present invention will now be described by way of example with reference to the accompanying drawings, in which:

Figure 1      shows a diagram of a joint;

Figure 2      shows a direction reference frame for a joint;

Figure 3      shows a pair of sensors fitted either side of a joint in accordance with some implementations;

Figure 4      shows a schematic diagram of a system for monitoring a joint;

Figure 5      is a diagram showing some of the functions of a system for monitoring a joint;

Figure 6      shows one of the sensors of figure 3 being removed;

Figure 7a    shows a graph of knee angle;

Figure 7b    shows a graph of a roll angle; and

Figure 8    shows a method in accordance with some implementations.

In the figures, like reference numerals indicate like parts.

DETAILED DESCRIPTION

The current subject-matter relates to a method for determining if a body sensor is in an unworn state and a system for implementing the method. The method is particularly directed to a system in which a pair of sensors is mounted either side of a joint, where the sensors are being used to monitor the angle of the joint over time. For example, if a person has injured the joint or had surgery on the joint, they may not be able to bend it through the full range of movement of a healthy joint. By monitoring the movement of the joint over time, a picture can be built up of the person's activity level and whether the range of movement of the joint is improving. If this information is to be useful, it is preferable for it to include only data obtained when the sensors are mounted in their intended position on the person. If data obtained when this is not the case is included in the information, this could skew the information such that it becomes less useful in monitoring the joint. Implementations of the present invention aim to avoid such skewing of the information.

The following describes specific examples of the use of sensors in relation to a knee joint on a human. However, the underlying principles are applicable to many different joints such as the ankle, elbow or wrist, and could also be applied to joints associated with other animals.

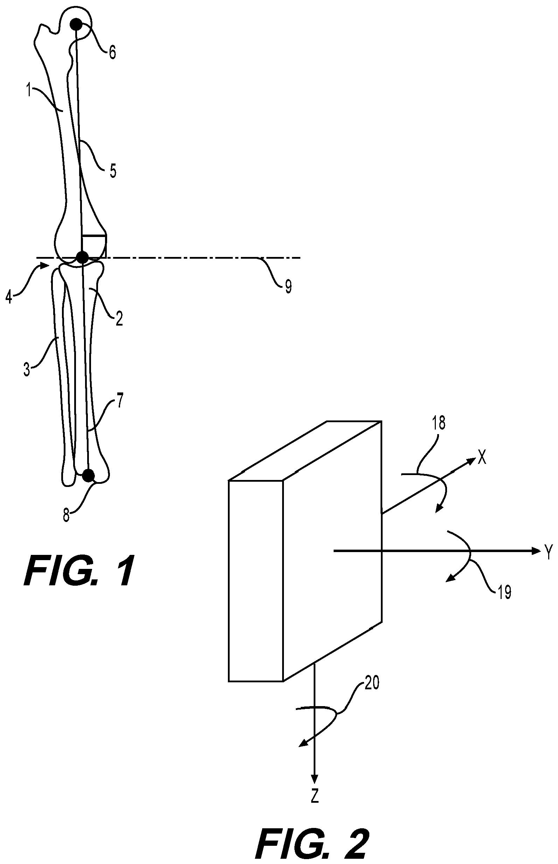

Figs. 1 and 2 are provided to allow a simple explanation of certain terms that are used within this specification. Fig. 1 illustrates a standard leg having a femur 1, a tibia 2 and a fibula 3. These are joined at a knee joint 4. The femur 1 defines a femoral mechanical axis 5 extending from the knee to a ball joint 6 which forms part of the person's hip. A tibial mechanical axis 7 extends from the knee 4 to the lower end 8 of the tibia 2 itself. The femur and the lower leg (made up of the tibia 2 and fibula 3) can pivot relative to each other about a knee joint axis 9. The femur and the lower leg thus define a plane in which the respective mechanical axes pivot relative to each other. Thus, each mechanical axis will substantially align with the respective part of the leg, such that the knee joint axis 9 is perpendicular to the plane in which the axes pivot. Thus the knee angle is thus typically the angle between the two mechanical axes.

This is an idealised situation, which forms the basic geometry considered by the present invention. It is possible to apply one or more compensation schemes to deal with any misalignment between the axes and the respective part of the leg.

Fig. 2 helps to define the coordinate system associated with the knee joint, as well as how the terms pitch and roll apply to the knee. The convention when discussing the knee joint is that, when a person is standing upright, the x-axis points forward i.e. away from the knee in the direction of walking, parallel to the ground, the y-axis points to the person's right, and the z-axis points downwards towards the ground. This convention applies to both left and right legs, i.e. the positive y-axis is always to the right hand side of the knee irrespective of the leg. The y-axis is therefore analogous to the knee joint axis 9.

The orientation of any sensors associated with the knee typically has two components. A rotation of the sensor about the x-axis is a roll motion, identified by an arrow 18, and defines a roll angle. A rotation of the sensor about the y-axis is a pitch motion, identified by an arrow 19, and defines a pitch angle. A third component, namely rotation of the sensor about the z-axis, would be a yaw motion, identified by an arrow 20, and would define a yaw angle.

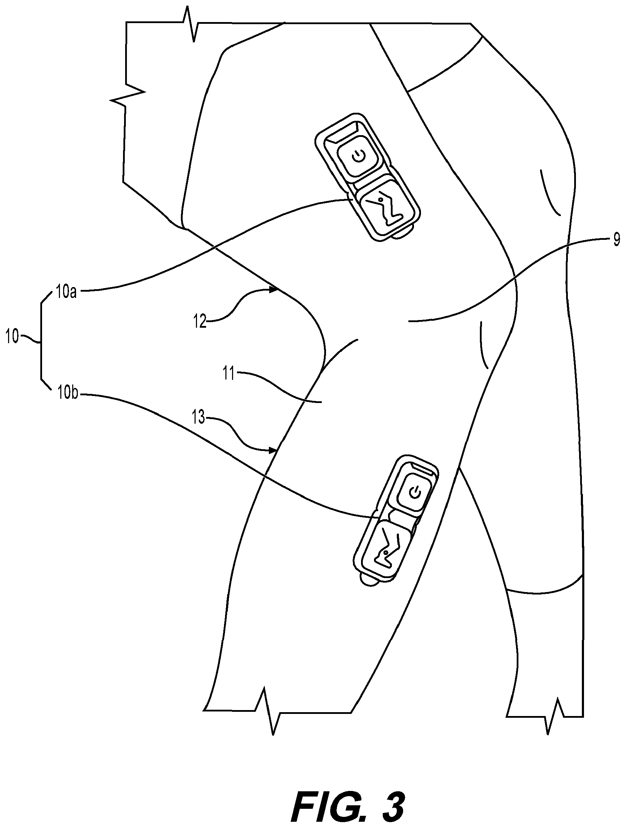

Fig. 3 illustrates a pair of sensors 10 attached to a leg 11. Each sensor contains one or more motion sensing devices which permit either (i) the pitch and/or roll of the individual sensor to be determined or (ii) the relative pitch and/or roll between the sensors to be determined. These motion sensing devices could be any suitable devices such as, but not limited to, an accelerometer, gyroscope, or a pair of strain gauges.

An upper sensor 10a is placed on the thigh 12 and a lower sensor 10b is placed on the calf 13. The purpose of the sensors is to monitor the flex of the knee at the knee joint, i.e. a pitch angle about the y-axis/knee joint axis 9. If the two sensors 10a, 10b could be aligned such that the z-axis of the sensor was parallel to the respective mechanical axis of the leg, and the sensor y-axis was parallel with the knee joint axis 9, the calculation of the knee angle would be a simple subtraction of the calf pitch angle from the thigh pitch angle. In practice, there may be a misalignment with the femoral and tibial mechanical axes which needs to be corrected for in order to obtain an accurate knee angle measurement.

In the example of the patient having had a total knee replacement or indeed any other knee surgery or knee complaint which results in limited movement of the knee, it can be helpful for a healthcare professional, or even the patient themselves, to monitor the knee angle over a lengthy period of time such as weeks or even months. Thus, a further problem can arise as the sensors will need to be removed periodically for numerous reasons including but not limited to cleaning of the sensor, recharging the sensors, increasing the comfort of the patient at night or cleaning of the patient in the sensor locations. When the sensors are removed or indeed if they fall off or become dislodged from the position in which knee angle has been being monitored, the sensors are said to be in an unworn state. It would be beneficial to know if one or both of the sensors has entered an unworn state, because this indicates that the sensor system of which they form part is in an unworn state. Thus this can be taken account of when presenting data for the monitoring. Systems and methods for detecting an unworn state are described in the following.

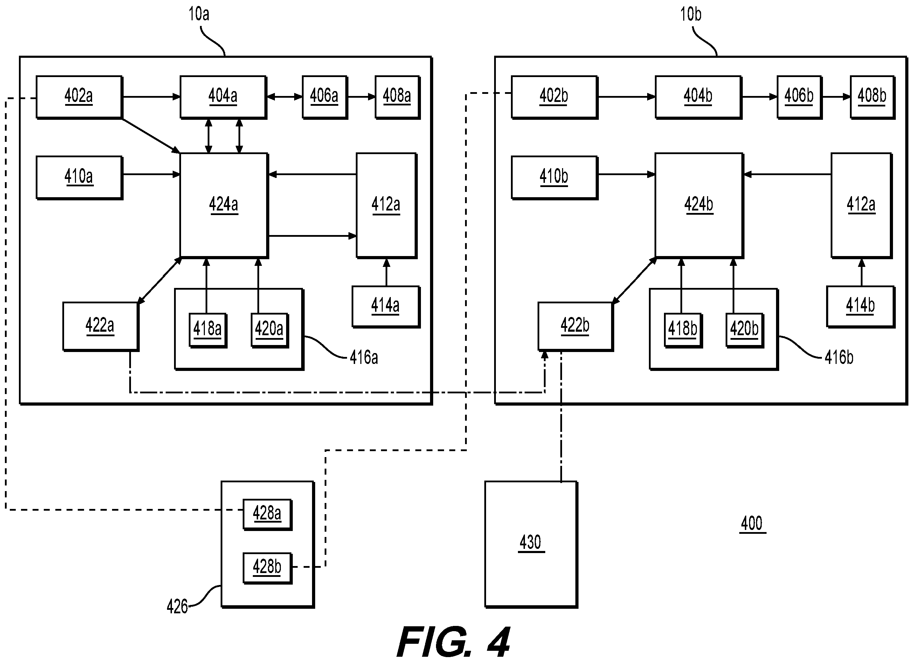

Fig. 4 illustrates schematically a sensor system 400 which includes the knee sensors 10, some components of the knee sensors 10 and operational connections to and from the sensors, and components thereof. It will be appreciated that the sensors 10 may each form part of a sensor unit configured to be mounted on a body part on a side of a joint as described with reference to Fig. 3. Thus each sensor 10 may form part of or be attached to some sort of patch that can be applied to skin or may be used in conjunction with an adhesive or other attachment means to enable mounting of the sensor to a body part.

The two sensors 10a, 10b comprise identical or similar components, which in each case are denoted by a same reference numeral and an a or b respectively. These components are: a power receiver/voltage regulator 402 connected to a cell charger and monitor 404, connected to a cell 406 (e.g. a Lithium-ion cell or other suitable energy storage device), connected to power supplies and support circuitry 408; a temperature sensor 410, which may in practice include any of multiple temperature sensors e.g. an external temperature sensor to sense temperature of skin to which the sensor unit is mounted, one or more internal temperature sensors to sense temperature of one or more components of the sensor itself; a power control/push switch logic unit 412 connected to an on/off push switch 414; an Inertial Measurement Unit (IMU) 416 which includes an accelerometer 418 and a gyroscope 420; and a Bluetooth Low Energy (BLE) module 422. All of the above-discussed components are connected to a microcontroller 424. The aforementioned on-sensor connections are shown with solid line arrows. It will be appreciated that further components may be present in the sensors 10 such as a magnetometer (to determine absolute movements and orientations of the patient), visual indicators of status etc., but these are omitted for clarity.

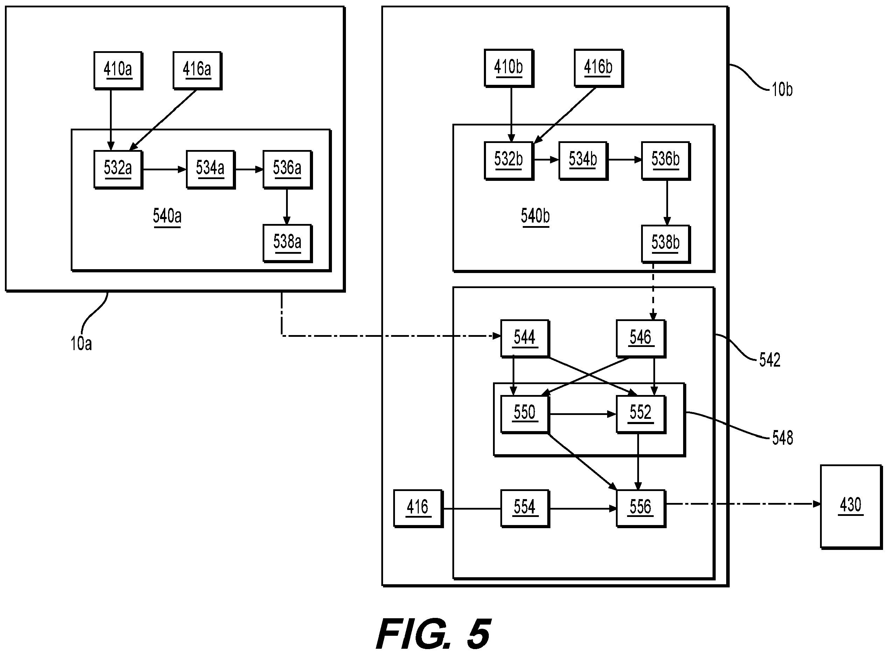

The sensors 10 have additional external connections. The power receiver/voltage regulators 402 can be connected to a charging unit 426. For example, they may be set into respective docking ports 428 for charging when required (dotted lines indicate these connections). The two BLE modules 422 can communicate wirelessly. The BLE module 410a of the sensor unit 10a may transmit data to the BLE module 422b of the sensor unit 10b, in implementations in which the sensor unit 10b acts as a master unit. This functionality will be described in more detail below with reference to Fig. 5. The BLE module 422b of the sensor unit 10b may transmit data to a mobile phone 430 or other device on which information pertaining to monitoring of the knee or other joint can be displayed, for example by interacting with an app running on the mobile phone 430. Such an app may be available for download and installation and may be specifically for interacting with the sensors 10 and to enable viewing of information relating to the joint being monitored. These transmissions are indicated by chained lines.

In operation, the accelerometers 418a,b and the gyroscopes 420a,b of the IMU 416 can take measurements relating to orientation and changes in orientation of their respective sensors 10 and movements that their respective sensors 10 undergo as a result of movement of the body parts to which they are attached. These movements reflect the use of the knee joint by the person during a time period in which the sensors 10 are attached. Together if desired with other sensed information such as readings provided by the temperature sensor(s) 410a, b, information calculated from these measurements can be provided to indicate the function of the knee over the time period. Other desired information can be indicated in conjunction with the knee function information e.g. knee temperature. In particular, it is possible to calculate the knee angle from accelerometer and gyroscope measurements, for example by calculating pitch and roll angles undergone by the sensors 10, and thus a picture of variation of the knee angle over a time period such as a day can be determined. One way to calculate knee angle using measurements from an accelerometer and a gyroscope is discussed in a paper from the 2011 IEEE International Conference on Rehabilitation Robotics entitled "Estimation of IMU and MARG orientation using a gradient descent algorithm" by Madgwick et al., the contents of which are herein incorporated by reference. Those skilled in the art will appreciate that other methods and algorithms could be used for this purpose. Moreover, the skilled reader will appreciate that other types of measurement equipment that can determine roll and pitch between two points could be used in place of the IMU 416.

Turning to Fig. 5, some functions of the sensor units 10 which facilitate the above-mentioned recording of data and performing of calculations will now be explained. Fig. 5 shows schematically some of the functions of the first and second sensors 10a, 10b. It will be understood that the functions shown and described in the following are not an exhaustive list of all the functions that may be implemented by the sensors and that the functional blocks shown are a simplified representation of some actual functions that sensors such as the sensors 10a, 10b may perform for the purpose of monitoring a joint. In a similar manner to Fig. 4, functional blocks that are common to both the sensors 10a, 10b are indicated by like reference numerals together with a sensor denotation a or b.

Both sensors 10a, 10b share some common functionality. The temperature sensor(s) 410 and the IMUs 416 are indicated by way of some measurement functions that the sensors 10a, 10b may perform. The raw measurements made by the temperature sensor(s) 410 and the IMUs 416 are provided to a data conversion unit 532, which processes the raw measurements. The processed measurements may be passed to a calibration unit 534, which can hold information relating to a calibration of the sensor units 10 which could have been performed previously, including corrections for any misalignment with the femur and tibia. Such a calibration may be updated periodically. The processed and calibrated data can be passed to an orientation estimation unit 536, which determines the orientations of the sensors 10. In some implementations, this includes the pitch and roll undergone by the sensors 10. The orientation estimation unit 536 may make use of methods such as the ones discussed above with reference to Fig. 4. The calculated parameters such as pitch and roll data can be passed to a transmitter such as an orientation data packing unit 538 for onward transmission. Such onward transmission could be by wired connections or unwired connections such as Bluetooth transmission or radio transmission etc.. The data conversion unit 532, the calibration unit 534, the orientation estimation unit 536 and the orientation data packing unit 538 can be considered to provide a functionality termed generally a sensing algorithm unit 540.

It can be seen in Fig. 5 that the second sensor 10b, which was shown in Fig. 3 mounted on the calf 13 of a patient, has some further functionality not present in the first sensor 10a, which was shown in Fig. 3 mounted to the thigh 12 of a patient. In this implementation, the second sensor 10b is a master sensor and is configured for mounting in a lower position on the patient than the first sensor 10a. It will be appreciated that the master sensor could instead be the first sensor 10a applied to the thigh of a patient and hence be configured to be mounted in an upper position to the second sensor 10b. The master sensor may be termed the fusion node and the other sensor may be termed the source node.

The further functionality present in the second sensor 10b can generally be termed a metrics algorithm unit 542. Within the metrics algorithm unit 542 are various functionalities discussed in the following. There is a receiver such as a first orientation data unpacking unit 544 arranged to receive data from the orientation data packing unit 538a of the first sensor 10a; a receiver such as a second orientation data unpacking unit 546 arranged to receive data from the orientation data packing unit 538b of the second sensor 10b. There is a calculation unit 548 which includes a knee angle estimation unit 550 and a worn classification unit 552. Both the knee angle estimation unit 550 and the worn classification unit 552 can receive data relating to angle of the sensors 10 from the first and second orientation data unpacking units 544, 546. The worn classification unit 552 can additionally receive an output from the knee angle estimation unit 550. The IMU 416 is functionally indicated again as providing an input to the metrics algorithm unit 542, specifically into a step count unit 554. The knee angle estimation unit 550, the worn classification unit 552 and the step count unit 554 are all functionally connected to an Activities of Daily Living (ADL) unit 556, which can transmit data to the mobile telephone 430 or other display device. This transmission is indicated by a chained line.

In operation, once mounted in position on a person's leg 11, the sensors can start taking measurements. One option for triggering this process is for the switches 414 to be used to switch on the sensors 10. Another additional or alternative option is for one or both of the sensors 10 to start interacting with the above-mentioned app on the phone 430. The temperature sensor(s) 10 can commence taking temperature readings. The accelerometers 418 and the gyroscopes 410 of the IMUs 416 can commence taking orientation information of the sensors 10, which may include information relating to changes in roll, pitch and, if desired, yaw angle. It is convenient for these measurements to be taken periodically so that multiple measurements can be taken over a time period in which the sensors 10 are being worn. For example, measurements may be taken 50 times/s i.e. at 50 Hz, or at some other suitable interval. The measured data passes through the various functional units common to the two sensors 10 until eventually the orientation data packing units 538 have processed it into a transmissible form. Following delivery to the metrics algorithm unit 542 within the second sensor 10b, specifically into the orientation data unpacking units 544, 546, the various functional blocks of the metrics algorithm unit 548 can process the data along with temperature data. The knee angle estimation unit 550 can use this data to calculate the knee angle at at least some of the times at which measurements were taken. The worn classification unit 552 can calculate the roll and pitch angles of the sensors 10 at at least some of the times at which measurements are taken. These calculations are then correlated in the ADL unit 556. This unit stores a summary of the metrics calculated from the obtained measurements, which may include any of the following: knee angle, step count, active time, load bearing time, and time worn. The knee angle could be stored as a value for each time measurements are taken but in practice, in order to provide a more useful output, this data may be correlated over the time period. For example, it may be stored as a histogram in 5 degree buckets in order to show the time spent in each bucket. Other possibilities for presenting the knee angle data will occur to the skilled reader. Depending on the memory capacity of the ADL 556, it may be preferable to store only the correlated data and not the raw measurements. The ADL unit 556 can operate to store data during the entirety of the time period in which the sensors 10 are worn, thereby giving an indication of the function of the knee during the patient's daily activities. This data can be uploaded to the mobile phone 130 when desired e.g. upon request or at the end of the time period. The data could also be further correlated over multiple time periods e.g. over the course of a week. If desired, the data may also be correlated over a specific time period within the time period in which the sensors are worn, for example during a period of exercise. A shorter burst of data such as this could be streamed to the mobile phone 130. Any data uploaded to the mobile phone 430 could be processed as necessary and viewed via the app.

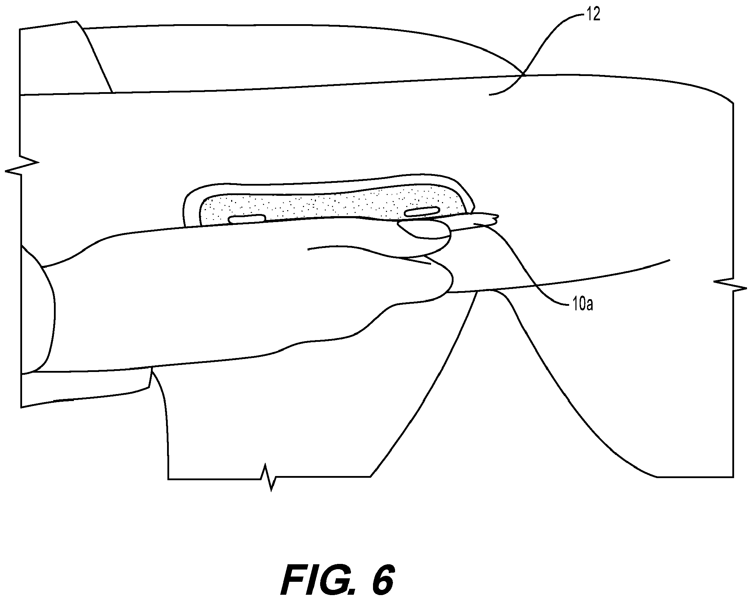

As noted above, it is undesirable to present data from measurements that are taken when one or both of the sensors 10 is not mounted in its intended position, because such data could be misleading as to the functionality of the knee. In order to address this issue, the present system includes a not worn detection system. With reference to Fig. 6, it will be appreciated that if one of the sensors 10 is removed, its orientation changes dramatically. This fact is used in the present system to enable detection of such removal. The systems and methods described in the following may be used after a previous determination that the sensors are worn e.g. after operation for any length of time as described above. Such systems and methods could also be used to determine that one or both of the sensors 10 has not yet been mounted but subsequently that they are both mounted on the leg 11 and could thus be used instead of provision of the on/off switches 414 in some circumstances. Alternatively, such systems and methods could be used as an alternative to waiting for interaction with the app on the mobile phone 430. As another alternative, such systems and methods could be used if the on/off switches 414 are used to switch on the sensors before they have been correctly mounted on the patient, so as not to take account of any measurements made before mounting is complete. They could also be used if one or both of the sensors 10 becomes dislodged from its intended position or falls off completely or to some extent. They may be used after an initial calibration of the sensors. They may also be used after an initial set up of the sensors. They could also be used after determining whether the sensors are correctly placed or that there is an error in their placement which can be corrected for. For example, as noted above, the first and second sensors 10 may include different functionality and each may be configured to be placed on a particular side of a joint in a particular orientation. Thus such systems and methods could be used to determine whether the sensors have been mounted on their correct respective body parts. Any of the possibilities discussed here could be used in any combination to determine that one or both of the sensors is not mounted and hence that the system is in an unworn state. Such systems and methods will now be described.

Referring back to Fig. 6, the exemplary first sensor 10a is being removed from the thigh 12 by peeling such that the sensor 10a undergoes a tilt. The exemplary tilt shown is a roll movement. It will be appreciated that such a movement will be at odds with the recorded movement of the other, second sensor 10b mounted on the calf 13, if the second sensor 10b is still mounted on the calf 13. This mismatch in relative orientation of the two sensors 10 can be used to determine that the sensor system 400 is in an unworn state. Even if the second sensor 10b is subsequently removed, the relative orientations of the two sensors will be different from their relative orientation when mounted across the patient's knee. This will also be the case if the second sensor is removed prior to removal of the first sensor. This will also be the case if either of the sensors is removed in a different manner e.g. by peeling with a different tilting motion such as yaw motion or by some combination of roll and pitch motion. Furthermore, the knee angle as calculated using the pitch and roll of the sensors 10 will not return a feasible knee angle in any of these situations. In other words, the observed pitch and/or roll angles and/or knee angle can be used to indicate a relative position of the body parts to which the sensors 10 are configured to be mounted i.e. in this case the thigh and calf, which is impossible or "inhuman".

An exemplary algorithm to determine an unworn state will now be described. At each time step at which measurements are taken, the worn classification unit 552 receives the current knee angle and roll angle of both sensors. Using these angles a "penalty" is calculated which is a measure of how inhuman the angles are. This penalty is calculated as follows:

a Knee, aRoll_F, aRoll_S in degrees where

> k = a Knee (knee angle)
> aRoll_F = roll angle of the second sensor 10b (where F indicates fusion sensor)
> aRoll_S = roll angle of the first sensor 10a (where S indicates source sensor)
> r = |aRoll_F - aRoll_S|
> p_k = knee angle penalty
> p_r = roll angle penalty
> p = penalty

The following conditional determinations are made:

$$p\_k = 0 \text{ if } -10 <= k <= 150$$

$$= 0.5 * ((k + 10) / 160)^4 \text{ if } k < -10$$
$$= 0.5 * ((k - 150) / 160)^4 \text{ if } k > 150$$

A feasible human knee angle is anywhere between approximately 10 degrees and approximately 150 degrees so if the angle is within this range, the penalty is zero, but if it is outside this range there is a penalty.

$$p\_r = 0 \text{ if } r <= 40$$
$$= 0.5 * ((r - 40) / 140)^4 \text{ if } r > 40$$

Thus up to 40 degrees of relative roll between the thigh 12 and the calf 13 is considered feasible and the penalty is zero, but above this amount is considered unfeasible or inhuman and there is a penalty.

It should be noted that the parameters chosen for the above calculations can affect the sensitivity of the system to inhuman orientations. The present applicant has found that the more sensitive the system is set, the greater the risk of misclassification of worn/unworn state. The above parameters are shown by way of example only as ones that the applicant has determined to be advantageous following significant experimentation. Generally speaking, the parameters may be chosen such that false readings do not occur but unworn states are not missed.

$$p = p\_k + p\_r$$

Thus a combination of the roll penalty and the knee angle penalty is used to determine whether the sensor system 400 is in an unworn state.

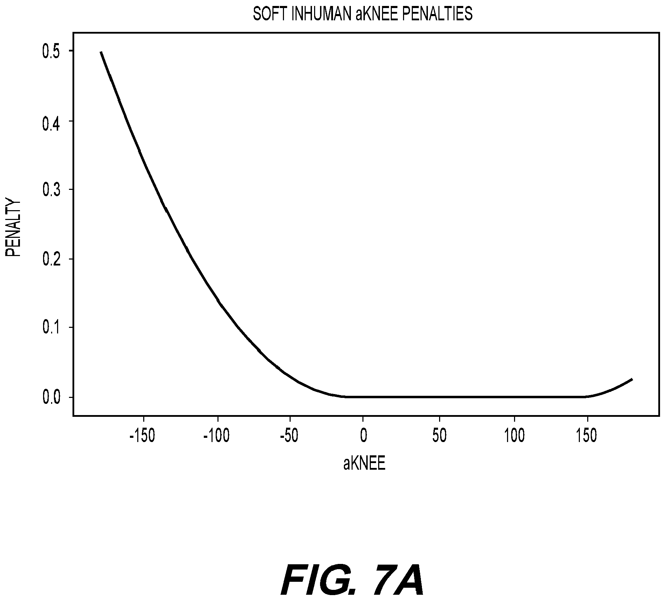

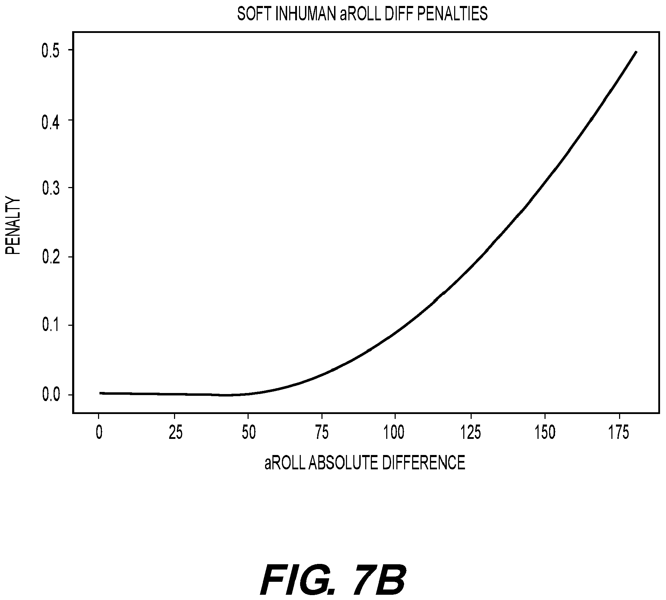

Figs. 7a & 7b show a graph of knee angle and roll angle penalties respectively as a function of the input angles. In this exemplary implementation, the final penalty is calculated as a sum of these two penalties, but a different mathematical combination of the knee and roll angle penalties could be used. The final penalty is then processed into a moving average (e.g. as an exponential recursive filter with forget rate of 0.05), which smooths the value of the final penalty over time. If this moving average goes over a threshold of 0.005, this indicates an unworn state.

Upon detection of an unworn state as described above, the sensor units, for example the metrics algorithm unit 548, can switch to a not worn state in terms of how they function. As discussed above, this is because in this situation, any data collected by the IMUs 416 will be "rogue" data, which is not indicative of the patient moving their knee. Thus in particular, it is desirable for a different procedure than the normal operation described above with respect to Figs. 4 & 5 to be adopted as regards calculation of knee angle at times subsequent to detection of the unworn state, because the picture built up over time of the patient's knee angle is an important indicator of knee health and so the system can operate to avoid skewing this data with rogue data calculated using measurements from an unworn sensor or sensors. There are various options for enabling omission of data subsequent to detection of an unworn state from being included in the information provided by the ADL unit 556 and in particular the correlated knee angle information. One option is to stop the accelerometers 418 and the gyroscopes 420 from collecting data. Another option is for the data not to be passed to the data conversion units 532, but to instead be discarded. Alternatively the data could not be transmitted from the orientation data packing units 538 or it could be transmitted but discarded upon receipt by the orientation data unpacking units 544, 546. Other options include that the knee angle is not calculated by the knee angle estimation unit 550 or that the knee angles are calculated at subsequent times but either the worn classification unit 552 does not pass on the data or the data is passed on but not included by the ADL unit 556 in its correlation process. The worn classification unit 552 could indicate the not worn state to the ADL 556 in various ways that will occur to the skilled reader.

One advantage of the present system and method is that the unworn state is detected without human intervention. Therefore, it is not reliant on a person having to remember to take any action to put the sensors into the unworn state.

After an unworn state of a sensor has been detected, alternative calculations may be made as part of the different procedure. For example, the ADL 556 could log a time duration for which the sensor system 400 is in the unworn state. This could be determined from the absence of data received or, if the data continues to be received, until the worn classification unit 552 indicates that the sensor(s) is no longer in an unworn state. In some implementations, setting the sensors 10 to charge on the charger 426 can trigger an unworn state and thus similar principles could be used to measure duration of time for which the sensors are charged. If the calculated penalty falls back to below the threshold, the sensor system 400 can return to normal operation. In some implementations, the sensor system 400 is set to await a user input before recommencing normal operation e.g. operation of the on/off switches on the sensors 10 or the master sensor 10b interacting with the app on the mobile telephone 430. In this way, the system 400 will only recommence normal operation when the sensors 10 have been placed in position across the joint. In other implementations, an automatic return to normal operation could be implemented when the calculated penalty falls back to below the threshold, but it will be appreciated that it is possible for the two sensors 10a, 10b to be disposed relative to each other as they would be when fitted across a joint, even when not actually fitted to a joint. Thus in order to prevent normal operation recommencing in this situation, some further information would need to be gathered e.g. detection of skin contact.

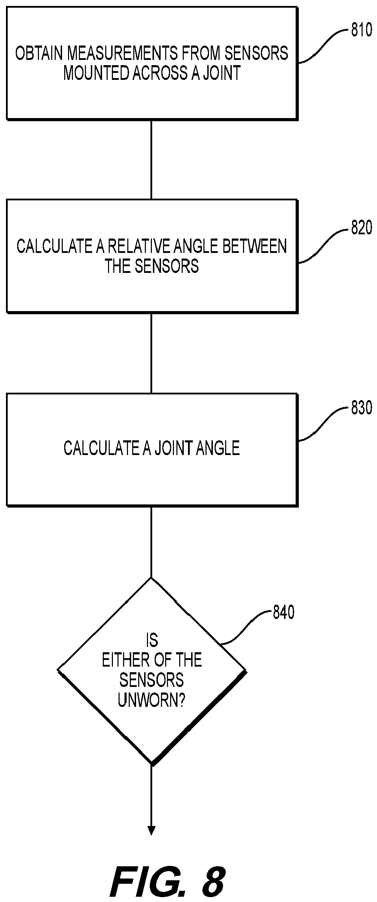

Fig. 8 shows a flowchart of a method in accordance with implementations of the invention. At 810, measurements are obtained from a pair of sensors mounted across a joint. For example, these could be the sensors 10 mounted on a thigh and calf across a knee joint of a patient.

At 812, the obtained measurements are used to calculate a relative angle between the sensors. In examples discussed above, this is performed by the worn classification functional unit 552. This could be an absolute difference in angle about any of the roll, pitch or yaw axes. In examples described above, the angle is roll. This calculation is simplified by the sensors 10 having both been placed on a same side of the leg 11 such that both lie substantially in the plane of the knee angle.

At 814, the obtained measurements are used to calculate a joint angle. In examples discussed above, this is performed by the knee angle estimation functional unit 550. In examples described above, the measurements may have been used to calculate roll and pitch angles and these values can be used to calculate the knee angle. Thus the obtained measurements are used indirectly to calculate the knee angle.

At 816, the relative angle and knee angle calculated are used to determine whether either of the sensors is unworn. In the examples discussed above, this determination could be made in the worn classification functional unit 552, which has determined roll angle at 812 and which can receive knee angle from the knee angle estimation functional unit 550. It could alternatively be made in the ADL unit 556 or by a separate functional unit.

As discussed above, if an unworn state is detected, various actions can be taken by the system to vary the procedure adopted subsequently with regard to data provided about the knee. As also discussed above, a subsequent return to normal operation could be implemented.

It will be appreciated that many variations to the above-discussed examples could be made without departing from the principles set out above and in the appended claims. For example, the functional units could be arranged differently and the calculations performed in a different order or by means of variations in the exemplary methods described above. The sensor components described above with reference to Fig. 4 could be different types or be used in different combinations and some of the described components may be provided as a single component. Whilst many examples above use relative roll angle between the two sensors 10, this is not essential and relative pitch or relative yaw could instead be used. Also, as mentioned above, in some implementations, mounting of the sensors on the wrong limbs can trigger an unworn state of the system, because doing this would result in a knee angle indicating that the knee is bending the wrong way. However, as an alternative to triggering an unworn state in this scenario, a different state could be triggered, the actual mounting position determined and taken into account during processing of the data in otherwise normal operation. In some implementations, the unworn detection system could be disabled prior to normal operation of the sensors, and a different procedure used to determine that the sensors are on the wrong limbs, to thereby trigger the different state. The unworn detection system could then be enabled so that subsequent removal etc. of the sensors would be detected.

In other variations, more than two sensors could be used. For example, it may be desirable to use three sensors if the joint under surveillance is a ball and socket joint which has three degrees of freedom of movement. In some cases, it may be desirable to use an additional sensor on a joint such as a knee joint to assist in determining orientations of the thigh and calf. For example, two sensors could be placed on one of the user's limbs. Measurements from such a third sensor could be processed in a similar manner to the processing described above for two sensors. One possibility is to process the data from the two sensors placed on one limb to obtain a set of data for that limb, and then process that in conjunction with the data from the other limb as described above.

The functions of the microcontroller 424 described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor such as the microcontroller 424, which can be special or general purpose, coupled to receive data and instructions from, and to transmit data and instructions to storage devices such as sticks. Such computer programs, which can be software, software applications, applications, components, or code, include machine instructions for a programmable processor such as the microcontroller 424, and can be implemented in a high-level procedural and/or object-oriented programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor and which can receive instructions as a machine-readable signal. Such a machine-readable medium can store instructions transitorily or non-transitorily.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features. In view of the foregoing description it will be evident to a person skilled in the art that various modifications may be made within the scope of the invention.

The present disclosure also provides for the following Examples:

    1. A method for determining an unworn state of a sensor system comprising a pair of sensors configured for mounting on first and second body parts either side of a joint, the method comprising:

        obtaining one or more measurements from each sensor;

calculating a relative angle between the two sensors using one or more of the obtained one or more measurements;

calculating, using one or more of the obtained one or more measurements, a joint angle between the first and second body parts, where the joint angle is defined in a plane of normal bending of the joint; and

determining, based on the calculated angles, whether either or both of the sensors is not mounted and if so, determining that the system is in an unworn state.

2. The method of Example 1, wherein obtaining one or more measurements from each sensor is performed multiple times in a time period and the calculating a relative angle and the calculating a joint angle is performed using measurements obtained at at least some of the multiple times, the method comprising the further step of, following a determination that the sensor system is in an unworn state, adopting a different procedure with regard to calculating the joint angle at subsequent times.

3. The method of Example 2, wherein adopting a different procedure comprises any one or more of: not calculating the joint angle at subsequent times; differently processing the calculated joint angles at subsequent times from those calculated at times prior to the determination; not storing the joint angles calculated at subsequent times.

4. The method of Example 2 or Example 3, further comprising correlating the joint angles and relative angles calculated at multiple times to provide information about the joint over the time period, and wherein adopting a different procedure comprises omitting any data calculated using measurements made at subsequent times from the information.

6. The method of Example 5, wherein the information comprises any one or more of: joint angle variation during the time period; duration of time in which the joint is active; duration of time in which the joint is bearing a load; length of the time period; and if the joint is a knee, step count over the time period.

7. The method of Example 5 or Example 6, wherein adopting a different procedure comprises performing calculations to provide different information from the information about the joint, wherein the different information includes any one or more of: unworn time; and if the first sensor is determined to be charging, charging time.

8. The method of any preceding Example, wherein an unworn state includes any one or more of: the first sensor having been at least partially removed from its mounted position; the first sensor falling off its mounted position at least to some extent; the first sensor being switched on but not mounted on the first body part; the second sensor being switched on but not mounted on the second body part.

9. The method of any preceding Example, wherein the determining if the first sensor is mounted on the first body part comprises calculating a value of a function of the calculated angles.

10. The method of Example 9, further comprising processing multiple calculated values of the function over a time period to determine a moving average value for the function and wherein the determining further comprises comparing the determined moving average value to a threshold value.

11. The method of Example 9 or Example 10, wherein the function comprises a function of a joint angle penalty and a relative angle penalty.

12. The method of Example 11, wherein if the joint angle is within a feasible range for the joint, the joint angle penalty is zero and if the joint angle is not within a feasible range for the joint, the joint angle penalty has a value which depends on how far outside the feasible range the joint angle is.

13. The method of Example 11 or Example 12, wherein if the relative angle is less than or equal to a threshold relative value, the relative angle penalty is zero and if the relative angle penalty is greater than the threshold relative value, the relative angle penalty depends on how much greater the relative angle is than the threshold relative value.

13. The method of any preceding Example, wherein the relative angle is a difference in tilt angle of the two body parts, where tilting occurs about an axis perpendicular or substantially perpendicular to the plane in which the joint angle is defined.

14. The method of Example 13, wherein the joint angle is defined in an x-z plane and the tilt angle is defined in the x-y plane and is non-zero if the first and second body parts undergo a relative roll about the x-axis.

15. The method of any preceding Example, further comprising, prior to the obtaining, determining whether the first and second sensors are mounted on correct respective first and second body parts and/or in substantially a predetermined orientation relative to the correct respective first and second body parts, and if one or both sensors is determined to be mounted on the wrong body part or in a different orientation from the predetermined orientation, adjusting the calculating steps to take account of an actual determined mounting location and/or orientation.

16. The method of any preceding Example, further comprising, prior to the obtaining, calibrating the first and second sensors with respect to a predetermined orientation relative to the respective first and second body parts.

17. The method of any preceding Example, wherein the joint is a knee or an elbow.

18. A system for providing information about a joint, comprising:
a first sensor unit configured to be mounted on a first body part on a first side of a joint, the first sensor unit comprising:

one or more first sensors arranged to take one or more measurements relating to the first body part and one or more measurements relating to the first sensor; and
a transmitter arranged to transmit taken measurements; and
a second, master sensor unit configured to be mounted on a second body part on a second side of a joint, the second master sensor unit comprising:

one or more second sensors arranged to take one or more measurements relating to the second body part and one or more measurements relating to the second sensor;
a receiver arranged to receive measurements transmitted from the first sensor unit transmitter; and
a calculation unit configured to calculate, using one or more of the measurements, a relative angle between the two sensors and a joint angle between the first and second body parts, where the joint angle is defined in a plane of normal bending of the joint, the calculation unit being further configured to determine, based on the calculated angles, whether either or both of the sensor units is not mounted and if so, determine that the system is in an unworn state.

19. The system of Example 18, wherein the first and second sensor units are configured to take measurements multiple times in a time period and the calculation unit is configured to calculate a relative angle and a joint angle using measurements obtained at each of at least some of the multiple times, the calculation unit being further configured to, following a determination that the system is in an unworn state, adopt a different procedure with regard to calculating the joint angle at subsequent times.

20. The system of Example 19, wherein the calculation unit is further configured to correlate the joint angles and relative angles calculated at multiple times to provide information about the joint over the time period, and wherein adopting a different procedure comprises omitting any data relating to measurements made at subsequent times from the information.

21. The system of Example 20, wherein the second, master sensor unit is configured to provide the information to a mobile device for viewing by a user.

22. The system of Example 20 or Example 21, wherein the information is for use in assessing health and/or rehabilitation of the joint.

23. The system of any of Examples 18 to 22, wherein the first and second sensor units are generally planar having front and back faces and are configured for their back faces to be mounted on the first and second body parts at a side of the joint substantially parallel to the plane of normal bending of the joint.

**Claims**

1.  A system (400) for providing information about a joint (4), comprising:

a pair of sensors (10a, 10b) configured for mounting on first (12) and second (13) body parts either side of a joint (4), wherein each sensor (10a; 10b) contains one or more motion sensing devices which permit either {i} the pitch and/or roll of the individual sensor (10a; 10b) to be determined or {ii} the relative pitch and/or roll between the sensors (10a, 10b) to be determined; and
a calculation unit configured to:

calculate a relative angle between the pair of sensors (10a, 10b) based on a first procedure;
determine, based on calculated angles, whether either or both of the sensor (10a, 10b) is not mounted and if so, determine that the system (400) is in an unworn state; and
calculate a relative angle between the pair of sensors (10a, 10b) based on a second procedure that is different from the first procedure when either or both of the sensor (10a, 10b) is determined to be in the unworn state.

**2.** The system of claim 1, wherein the either or both sensors (10a, 10b) are configured to take measurements multiple times in a time period in the first procedure and the calculation unit is configured to calculate a relative angle and a joint angle using measurements obtained at each of at least some of the multiple times, and wherein either or both sensors (10a, 10b) are configured to take fewer measurements over the same time period in the second procedure.

**3.** The system of claim 2, wherein the calculation unit is further configured to correlate the joint angles and relative angles calculated at multiple times to provide information about the joint over the time period, and wherein the second procedure comprises omitting any data relating to measurements made at subsequent times from the information.

**4.** The system of claim 3, configured to provide the information to a mobile device for viewing by a user.

**5.** The system of claim 3 or 4, wherein the information is for use in assessing health and/or rehabilitation of the joint.

**6.** The system of any one of claims 1 to 5, wherein the pair of sensors (10a, 10b) are generally planar having front and back faces and are configured for their back faces to be mounted on the first (12) and second (13) body parts at a side of the joint substantially parallel to the plane of normal bending of the joint.

**7.** The system of any one of claims 1 to 6, wherein the calculation unit is configured to determine a duration of time that the first and/or second sensor is in the unworn state.

**8.** The system of any one of claims 1 to 7, wherein the calculation unit is configured to determine whether the first and/or second sensor is charging time and if the first and/or second sensor is determined to be charging, determine the charging time.

**9.** The system of any one of claims 1 to 8, wherein determining if either or both of the sensor (10a, 10b) is determined to be in an unworn state comprises calculating a value of a function of the calculated angles.

**10.** The system of claim 9, wherein the calculation unit is further configured to process multiple calculated values of the function over a time period to determine a moving average value for the function and wherein the determining whether the sensor is in an unworn state further comprises comparing the determined moving average value to a threshold value.

**11.** The system of claim 9 or 10, wherein the function comprises a function of a joint angle penalty and a relative angle penalty.

**12.** The system of claim 11, wherein if the joint angle is within a feasible range for the joint, the joint angle penalty is zero and if the joint angle is not within a feasible range for the joint, the joint angle penalty has a value which depends on how far outside the feasible range the joint angle is.

**13.** The system of claim 11 or 12, wherein if the relative angle is less than or equal to a threshold relative value, the relative angle penalty is zero and if the relative angle penalty is greater than the threshold relative value, the relative angle penalty depends on how much greater the relative angle is than the threshold relative value.

**14.** The system of claim 10 wherein when the calculation unit is configured to transition from the second procedure to the first procedure when the determined moving average value is less than the threshold value.

**FIG. 1**

**FIG. 2**

**FIG. 3**

**FIG. 4**

EP 4 385 406 A2

**FIG. 5**

FIG. 6

EP 4 385 406 A2

SOFT INHUMAN aKNEE PENALTIES

FIG. 7A

EP 4 385 406 A2

SOFT INHUMAN aROLL DIFF PENALTIES

FIG. 7B

```
┌─────────────────────────────────┐
│ OBTAIN MEASUREMENTS FROM SENSORS │─── 810
│     MOUNTED ACROSS A JOINT       │
└─────────────────────────────────┘
                 │
┌─────────────────────────────────┐
│ CALCULATE A RELATIVE ANGLE BETWEEN │─── 820
│          THE SENSORS             │
└─────────────────────────────────┘
                 │
┌─────────────────────────────────┐
│      CALCULATE A JOINT ANGLE     │─── 830
└─────────────────────────────────┘
                 │
              ◇ IS
          EITHER OF THE  ─── 840
            SENSORS
            UNWORN?
                 │
                 ▼
```

## FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1915139 A